# EUROPEAN PATENT APPLICATION

(11) **EP 4 542 211 A1**
(43) Date of publication of application: **23.04.2025**
(21) Application number: 23849374.6
(22) Date of filing: 31.07.2023
(51) Int. Cl.: G01N 27/26, G01N 27/27, G01N 27/30, G01N 27/416, G01N 35/10, G01N 33/48, A61B 5/05

(54) **DETECTION ASSEMBLY FOR MEDICAL DETECTION EQUIPMENT, AND MEDICAL DETECTION EQUIPMENT**

(30) Priority: 31.07.2022 CN 202210912914
(71) Applicant: Edan Instruments, Inc., Shenzhen, Guangdong 518000 (CN)
(72) Inventor: HUANG, Gaoxiang, Shenzhen, Guangdong 518000 (CN); LI, Yuanbiao, Shenzhen, Guangdong 518000 (CN); ZHUANG, Zhenwei, Shenzhen, Guangdong 518000 (CN); ZHAO, Zhixiang, Shenzhen, Guangdong 518000 (CN)
(74) Representative: Herrero & Asociados, S.L.
(86) International application number: PCT/CN2023/110394
(87) International publication number: WO 2024/027667

(57) **Abstract**

The present application provides a detection assembly for medical detection equipment, and medical detection equipment. The detection assembly comprises a shell and a detection electrode. The shell is provided with a top surface, a bottom surface, at least one side surface, a liquid inlet, and a liquid outlet. The at least one side surface is separately connected to the top surface and the bottom surface. A cavity is formed in the shell. The liquid inlet and the liquid outlet are separately communicated with the cavity, the area of the top surface adjacent to the at least one side surface is recessed to form a groove, and the detection electrode is located in the groove. According to the present application, the detection electrode is arranged in the groove formed by recessing the area of the top surface and at least one side surface of the shell of the detection assembly, the plane where the detection electrode is located is parallel to the top surface, and the detection electrode is not located on a surface of the detection assembly, so that in the process of inserting the detection electrode in a slot, the edge of a slot opening is not in contact with the detection electrode of the detection assembly, the size of the slot opening is reduced, the size of the medical detection equipment is reduced, and the thickness of the detection assembly is also reduced.

## Description

### TECHNICAL FIELD

Embodiments of the present disclosure relate to the technical field of sample analysis, and in particular to a detection assembly for a medical detection device and a medical detection device.

### BACKGROUND

During a process of a sample detection, a detection assembly is required to be inserted into a slot of a medical detection device, enabling a detection electrode on the detection assembly to be in contact with a contact electrode in the slot of the medical detection device, thereby allowing a detection circuit in the medical detection device to receive signal transmitted from the detection assembly. Thus, the sample detection is achieved.

The detection assembly is substantially a box-shaped hexahedron. The detection electrode is disposed on a surface of the detection assembly. Therefore, during a process of the detection assembly being inserted into the slot of the medical detection device, to avoid a damage to the detection electrode due to an edge of a slot opening, i.e., an opening of the slot, being in contact with the detection electrode on the surface of the detection assembly, the slot opening is required to be slightly larger than a cross section of the detection assembly. In addition, the detection electrode protruding from the surface of the detection assembly may further increase an overall thickness of the detection assembly.

### SUMMARY OF THE DISCLOSURE

Some embodiments of the present disclosure provide a detection assembly for a medical detection device and a medical detection device to address the problem above.

Some embodiments of the present disclosure provide a detection assembly for a medical detection device. The detection assembly includes a housing and a detection electrode. The housing includes a top surface, a bottom surface, at least one side surface, a fluid inlet, and a fluid outlet. The at least one side surface is connected to each of the top surface and the bottom surface. A cavity is defined within the housing. Each of the fluid inlet and the fluid outlet is communicated with the cavity. A groove is recessed from an area of the top surface that is adjacent to the at least one side surface. The detection electrode is disposed in the groove. The groove defined on a part of the top surface has an opening defined on the at least one side surface.

Some embodiments of the present disclosure provide a medical detection device. The medical detection device includes a device body. The device body includes a slot and a contact electrode. The slot is configured to receive the detection assembly mentioned above. When the detection assembly is inserted into the medical detection device, the contact electrode is in contact with the detection electrode of the detection assembly.

Some technical effects of the present disclosure may be the following. The detection electrode is disposed in the groove defined by a recessed area of the top surface and a recessed area of the at least one side surface of the housing of the detection assembly together. The plane where the detection electrode is disposed is substantially parallel to the top surface. In other words, the detection electrode is not disposed on a surface of the detection assembly. Consequently, during a process of the detection assembly being inserted into the slot, an edge of a slot opening, i.e., an opening of the slot, is not in contact with the detection electrode of the detection assembly, thereby reducing a size of the slot opening, minimizing the volume of the medical detection device, and further reducing the thickness of the detection assembly.

It should be understood that, the general description above or the detailed description below is merely illustrative and interpretative and shall not limit the present disclosure.

### BRIEF DESCRIPTION OF THE DRAWINGS

The following provides a further explanation of the present disclosure in conjunction with figures and embodiments. The figures are as follows.
FIG. 1 is a schematic structural view of a detection assembly for a medical detection device according to some embodiments of the present disclosure.
FIG. 2 is a schematic structural view of a detection assembly for a medical detection device according to some other embodiments of the present disclosure.
FIG. 3 is a schematic structural view of a detection assembly for a medical detection device according to still some other embodiments of the present disclosure.
FIG. 4 is a schematic exploded view of a detection assembly according to some embodiments of the present disclosure.
FIG. 5 is a schematic top plan view of a second housing of a detection assembly according to some embodiments of the present disclosure.
FIG. 6 is a schematic structural view of a medical detection device according to some embodiments of the present disclosure.

### DETAILED DESCRIPTION

In the present disclosure, the term "embodiment" means that the specific features, structures, or characteristics described in connection with the embodiment may be included in at least one embodiment of the present disclosure. The term "embodiment" used in various locations of the specification does not necessarily refer to the same embodiment, nor does it imply a mutually exclusive independent or alternative embodiment. Those skilled in the art explicitly or implicitly understand that the embodiments described herein may be combined with other embodiments.

The term "and/or" in the present disclosure is merely a way of describing the relationship between associated objects, indicating three possible relationships. For example, "A and/or B" may represent: A exists alone, both A and B exist, or B exists alone. In addition, the character "/" herein generally represents an "or" relationship between associated objects before and after it. Furthermore, the term "plurality" herein means two or more. The term "at least one" refers to any one or a combination of at least two from a set. For example, "including at least one of A, B, and C" may refer to any one or more elements selected from the set of A, B, and C. In addition, the terms "first", "second", and "third" in the present disclosure are used merely for descriptive purposes and should not be understood to indicate or imply relative importance or to suggest the number of technical features indicated.

During a process of a sample detection, a detection assembly is required to be inserted into a slot of a medical detection device, enabling a detection electrode on the detection assembly to be in contact with a contact electrode in the slot of the medical detection device, thereby allowing a detection circuit in the medical detection device to receive signal transmitted from the detection assembly and the sample detection to be achieved.

The detection assembly is substantially a box-shaped hexahedron. The detection electrode is disposed on a surface of the detection assembly. To facilitate an avoidance of the detection assembly by the contact electrode of the medical detection device during the process of the detection assembly being inserted into the slot of the medical detection device, a driving assembly of the contact electrode is disposed in the medical detection device. The driving assembly is configured to, before the detection assembly is inserted into the slot, retain the contact electrode in an area that is inside the medical detection device but outside the slot. The driving assembly is further configured to, after the detection assembly is completely inserted into the slot, drive the contact electrode to move toward the slot until the contact electrode is in contact with the detection electrode on the surface of the detection assembly. In this case, a space inside the slot of the medical detection device is not effectively utilized before the detection assembly is inserted into the slot. Moreover, to avoid or provide an avoidance to the detection electrode on the surface of the detection assembly, an opening of the slot is required to be larger than a cross section of the detection assembly, thereby further increasing a volume of the medical detection device. In addition, the detection electrode protruding from the surface of the detection assembly may further increase a thickness of the detection assembly.

To solve the problem above, some embodiments of the present disclosure provide the following technical solutions. To assist those skilled in the art to better understand the technical solutions of the present disclosure, the following describes the technical solutions in further detail in conjunction with figures and embodiments.

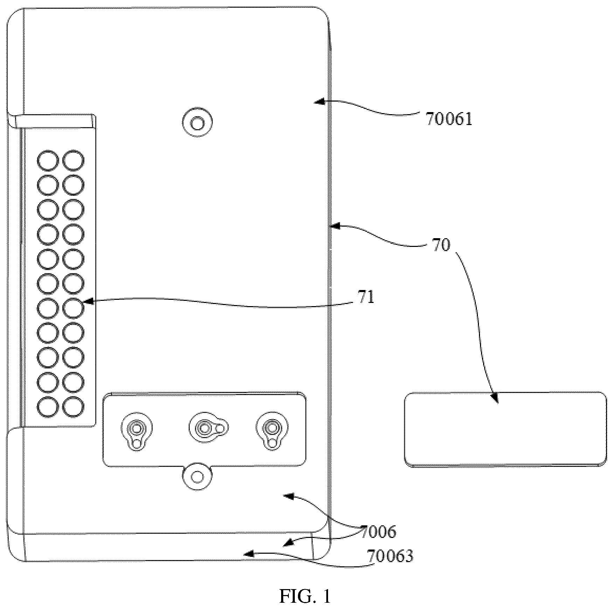

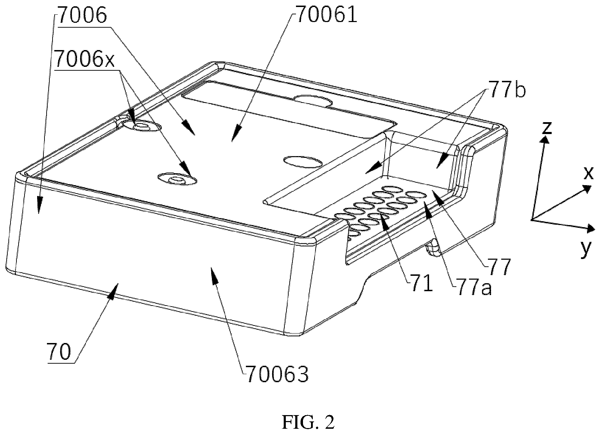

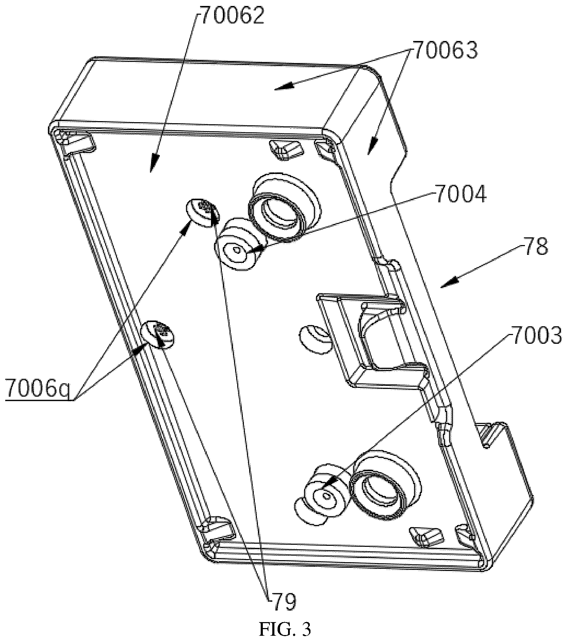

As shown in FIGS. 1-3, FIG. 1 is a schematic structural view of a detection assembly for medical detection device according to some embodiments of the present disclosure, FIG. 2 is a schematic structural view of a detection assembly for a medical detection device according to some other embodiments of the present disclosure, and FIG. 3 is a schematic structural view of a detection assembly for a medical detection device according to still some other embodiments of the present disclosure. The detection assembly 70 includes a housing 7006 and a detection electrode 71. The housing 7006 includes a top surface 70061, a bottom surface 70062, at least one side surface 70063, a fluid inlet 7003, and a fluid outlet 7004. The at least one side surface 70063 is connected to each of the top surface 70061 and the bottom surface 70062. A cavity 7006a is defined within the housing 7006. Each of the fluid inlet 7003 and the fluid outlet 7004 is communicated with the cavity 7006a. A groove 77 is recessed from an area of the top surface 70061 that is adjacent to the at least one side surface 70063. The detection electrode 71 is disposed in the groove 77. The groove 77 defined on a part of the top surface 70061 has an opening 78 defined on the at least one side surface 70063.

In above embodiments, the detection assembly 70 is substantially in shape of a cuboid. The detection assembly 70 includes the top surface 70061, the bottom surface 70062, and four side surfaces 70063. A top end of each of the four side surfaces 70063 is connected to an edge of the top surface 70061 and a bottom end of each of the four side surfaces 70063 is connected to an edge of the bottom surface 70062, thereby enclosing the cavity 7006a. The fluid inlet 7003 and the fluid outlet 7004 are defined on the bottom surface 70062. A reagent contained in a liquid package of a reagent kit may be transmitted to the cavity 7006a through the fluid inlet 7003. Waste liquid in the cavity 7006a may be discharged through the fluid outlet 7004 and then transmitted to a waste liquid bag of the reagent kit through a pipeline.

As shown in FIG. 2, an x-axis refers to a direction along a length of the top surface 70061 and the length of the top surface 70061 further represents a length of the detection assembly. A y-axis refers to a direction along a width of the top surface 70061 and the width of the top surface 70061 further represents a width of the detection assembly. A z-axis refers to a direction along a height of the at least one side surface 70063 and the height of the at least one side surface 70063 further represents a height of the detection assembly. A length direction, a width direction, and a height direction of the groove 77 correspond to a length direction, a width direction, and a height direction of the detection assembly, respectively. An area of the top surface 70061 and an area of one of the at least one side surface 70063 are recessed to define the groove 77. A depth of the groove 77 is less than the height of the detection assembly. A width and a length of the groove 77 are smaller than the width and length of the top surface 70061, respectively. The detection electrode 71 is disposed in the groove 77. The groove 77 defined on a part of the top surface 70061 has an opening 78 defined on the at least one side surface 70063.

In some embodiment, the groove 77 is defined by the top surface 70061 and three adjacent side surfaces 70063 together. The depth of the groove 77 is less than the depth of the detection assembly. The length of the groove 77 is equal to the length of the top surface 70061. The width of the groove 77 is less than a length of each of two side surfaces 70063 in opposite. In this case, the detection assembly 70 has a stepped shape. The detection electrode 71 is disposed in the groove 77. The groove 77 defined on a part of the top surface 70061 has an opening 78 defined on the at least one side surface 70063.

Therefore, in above embodiments, the detection electrode is disposed in the groove defined by the recessed area of the top surface and the recessed area of the at least one side surface of the housing of the detection assembly together. A plane where the detection electrode is disposed is substantially parallel to the top surface. In other words, the detection electrode is not disposed on a surface of the detection assembly. Consequently, during the process of the detection assembly being inserted into a slot, an edge of a slot opening, i.e., an opening of the slot, is not in contact with the detection electrode of the detection assembly, thereby reducing a size of the slot opening, minimizing the volume of the medical detection device, and further reducing the thickness of the detection assembly. Furthermore, during the process of the detection assembly being inserted into the slot, one of the at least one side surface where the groove is defined is inserted into the slot and is in contact with or abutted against a bottom of the slot first. In this case, a contact electrode of the medical detection device may be disposed in a space at the bottom of the slot. Thus, during the process of the detection assembly being inserted into the slot, the contact electrode no longer has to avoid the detection assembly and may be disposed in the space at the bottom of the slot, thereby effectively utilizing the space inside the slot before the detection assembly is inserted into the slot.

In some embodiments of the present disclosure, the top surface 70061 and the bottom surface 70062 are arranged in parallel. The number of the at least one side surface 70063 is four. The four side surfaces 70063 are sequentially connected to each other. The groove 77 and the opening 78 are defined on an area where the top surface 70061 is connected to one of the four side surfaces 70063. The width of each of the groove 77 and the opening 78 is smaller than the width of each of the top surface 70061 and the one of the four side surfaces 70063, respectively. A portion of the groove 77 corresponding to the top surface 70061 includes a plane 77a and a side surface 77b.The plane 77a is parallel to each of the top surface 70061 and the bottom surface 70062. The side surface 77b is perpendicular to the plane 77a and is connected to each of the top surface 70061 and the plane 77a. The detection electrode 71 is disposed on the plane 77a.

In above embodiments, an area of the top surface 70061 and an area of one of the at least one side surface 70063 are recessed to define the groove 77. The width and the length of the groove 77 are smaller than the width and the length of the top surface 70061, respectively. The depth of the groove 77 is smaller than the height of the detection assembly. The length and the width of the one of the opening 78 are smaller than the length and the width of the at least one side surface 70063, respectively. The groove 77 includes the plane 77a and the three side surfaces 77b. The opening 78 is defined by the plane 77a and the three side surfaces 77b. A top end of each of the three side surfaces 77b is connected to the edge of the top surface 70061 that surrounds the groove 77. A side end of each of two of the three side surfaces 77b is connected to an edge of the at least one side surface 70063 of the housing 7006 that surrounds the groove 77. The detection electrode 71 is disposed on the plane 77a of the groove 77. The length and the width of the opening 78 may be smaller than or equal to the length and the depth of the groove 77, respectively. In some embodiments, the length and the width of the opening 78 are equal to the length and the depth of the groove 77, respectively.

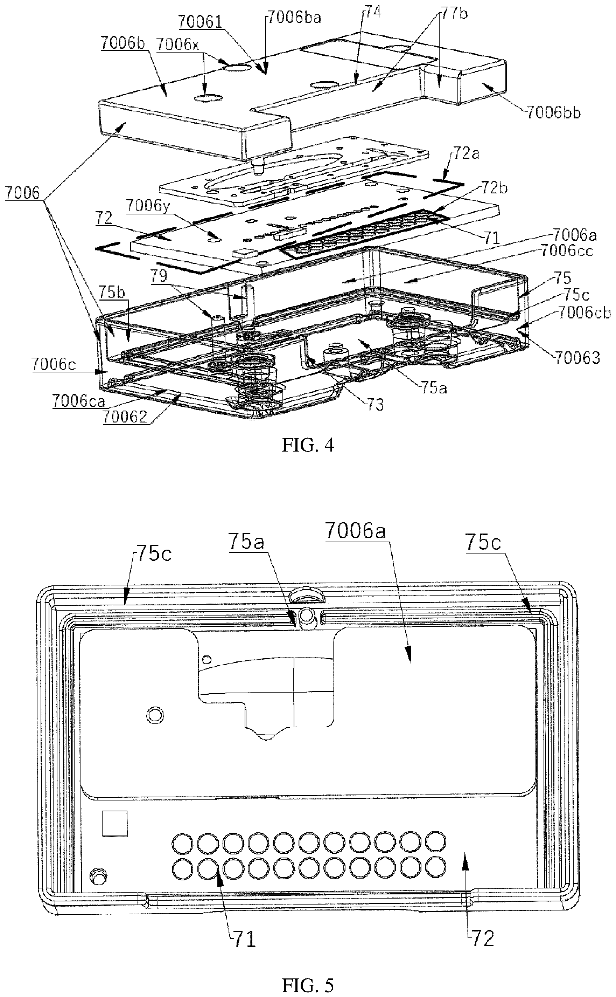

As shown in FIG. 4, FIG. 4 is a schematic exploded view of a detection assembly according to some embodiments of the present disclosure. As shown in FIG. 4, the housing 7006 includes a first housing 7006b and a second housing 7006c. The top surface 70061 is a surface of the first housing 7006b facing away from the second housing 7006c. The detection assembly 70 further includes a circuit board 72. The circuit board 72 has a first region 72a and a second region 72b. The detection electrode 71 is disposed on in the second region 72b. The second housing 7006c includes a base 7006ca and at least one first side plate 7006cb disposed around the base 7006ca. A bottom end of the at least one first side plate 7006cb is connected to an edge of the base 7006ca. A first notch 73 is defined on an end of the first side plate 7006cb away from the base 7006ca. An accommodating chamber 7006cc is defined by the base 7006ca and the at least one first side plate 7006cb. The bottom surface 70062 is a surface of the base 7006ca away from the first housing 7006b. The at least one side surface 70063 is an outer surface of the at least one first side plate 7006cb. The first housing 7006b cooperates with the at least one first side plate 7006cb to cover the accommodating chamber 7006cc. The circuit board 72 is disposed between the base 7006ca and the first housing 7006b. The first region 72a is located within the accommodating chamber 7006cc. The second region 72b is an area of the circuit board 72 that is outside the accommodating chamber 7006cc and corresponds to the groove 77, to form the plane 77a of the groove 77.

In above embodiments, the housing 7006 includes the first housing 7006b and the second housing 7006c. The first housing 7006b is sleeved on the second housing 7006c and covers the accommodating chamber 7006cc of the second housing 7006c, to form the cavity 7006a. The circuit board 72 is disposed between the first housing 7006b and the second housing 7006c. The detection electrode 71 is disposed on the second region 72b of the circuit board 72. The first region 72a of the circuit board 72 is located within the cavity 7006a. The second region 72b is located within the groove 77 and the opening 78, exposing the detection electrode 71 within the groove 77 defined by the recessed area of the top surface 70061 of the first housing 7006b and the first notch 73 of the at least one first side plate 7006cd of the second housing 7006c. The second region 72b of the circuit board 72 forms the plane 77a of the groove 77.

In some embodiments of the present disclosure, the first housing 7006b includes a cover 7006ba and at least one second side plate 7006bb disposed around the cover 7006ba. A top end of the at least one second side plate 7006bb is connected to an edge of the cover 7006ba. The top surface 70061 is a surface of the cover 7006ba away from the second housing 7006c. A second notch 74 is defined on an area of the cover 7006ba that is close to the first notch 73. One of the at least one second side plate 7006bb that corresponds to the second notch 74 extends along a contour of the second notch 74. The first notch 73, the second notch 74, the one of the at least second side plate 7006bb that corresponds to the second notch 74, and the second region 72b together define the groove 77. The one of the at least one second side plate that corresponds to the second notch forms the side surface 77b of the groove 77.

In above embodiment, the first housing 7006b includes the cover 7006ba and the at least one second side plate 7006bb. The top end of the at least one second side plate 7006bb is connected to the edge of the cover 7006ba. The second notch 74 is defined on the cover 7006ba. The one of the at least one second side plate 7006bb that corresponds to the second notch 74 is designed in the shape matching with the shape of the second notch 74. That is, the shape of the second region 72b may be connected to the shape of the at least one second side plate 7006bb in an edge-to-edge manner. The one of the at least one second side plate 7006bb that corresponds to the second notch 74 forms the side surface 77b of the groove 77. The second notch 74 is adjacent to the first notch 73 of the at least one first side plate 7006cb. Therefore, the first notch 73, the second notch 74, the one of the at least one second side plate 7006bb that corresponds to the second notch 74, and the second region 72b together define the groove 77.

In some embodiments of the present disclosure, the housing 7006 further includes a third housing 75. The third housing 75 includes a mounting plate 75a and at least one third side plate 75b disposed around the mounting plate 75a. A bottom end of the at least one third side plate 75b is connected to an edge of the mounting plate 75a. Each of the mounting plate 75a and the at least one third side plate 75b is disposed within the accommodating chamber 7006cc of the second housing 7006c. A top end of the at least one third side plate 75b is connected to a top end of the at least one first side plate 7006cb. The circuit board 72 is disposed on the mounting plate 75a and is located between the mounting plate 75a and the first housing 7006b. A third notch 76 is defined on one of the at least one third side plate 75b that corresponds to the first notch 73. The third notch 76 has a shape matching with a shape of the first notch 73.

In above embodiment, the third housing 75 is disposed within the accommodating chamber 7006cc of the second housing 7006c. The top end of the at least one third side plate 75b of the third housing 75 is connected to the top end of at least one second side plate 7006bb of the second housing 7006c. The bottom end of the at least one third side plate 75b is connected to the edge of the mounting plate 75a. The mounting plate 75a is substantially parallel to each of the base 7006ca and the cover 7006ba. The circuit board 72 is disposed on the mounting plate 75a. The third notch 76 is defined on one of the at least one third side plate 75b. The third notch 76 has the shape matching with the shape of the first notch 73. A height of the at least one third side plate 75b is slightly greater than a depth of the first notch 73. When the circuit board 72 is disposed on the mounting plate 75a, the horizontal plane 77a where an upper surface of the circuit board 72 is located is flush with or slightly higher than the horizontal plane 77a that is recessed to define the first notch 73. The second housing 7006c and the third housing 75 may be integrated into one piece or connected in a detachable manner.

As shown in FIG. 5, FIG. 5 is a top plan view of a second housing of a detection assembly according to some embodiments of the present disclosure. As shown in FIG. 5, a raised rib 75c is disposed on the edge of the mounting plate 75a that is close to the at least one third side plate 75b. The raised rib 75c is disposed around a periphery of the circuit board 72 to limit the circuit board 72.

In above embodiments, a shape enclosed by the raised rib 75c matches with a shape of the circuit board 72. The raised rib 75c is disposed around the periphery of the circuit board 72 to limit the circuit board 72, thereby avoiding the circuit board 72 from shifting within the accommodating chamber 7006cc and ensuring that the detection electrode 71 on the second region 72b of the circuit board 72 remains in a fixed position. In this way, when the detection assembly 70 is inserted into the slot, a misalignment between the contact electrode and the detection electrode 71 during a process of the contact electrode being in contact with the detection electrode 71 may be avoided.

In some embodiments, when the circuit board 72 is disposed on the mounting plate 75a, the horizontal plane 77a where the upper surface of the circuit board 72 is located is flush with or slightly above the raised rib 75c.

In some embodiments of the present disclosure, the detection assembly 70 includes at least one fixing member 79. A first through hole 7006x is defined on the cover 7006ba of the first housing 7006b, a second through hole 7006y is defined on the circuit board 72, a third through hole (not shown in the figure) is defined on the mounting plate 75a, and a fourth through hole 7006q is defined on the base 7006ca. The at least one fixing member 79 penetrates through the fourth through hole 7006q, the third through hole, the second through hole 7006y, and the first through hole 7006x, to fix the first housing 7006b, the circuit board 72, and the second housing 7006c to each other.

Therefore, in above embodiments, the number of the at least one fixing member 79 is two. The two fixing members 79 penetrate through the through hole on each of the cover 7006ba, the mounting plate 75a, and the base 7006ca, to fix the first housing 7006b, the circuit board 72, and the second housing 7006c to each other.

In some embodiments of the present disclosure, a height of the at least one second side plate 7006bb is greater than or equal to one-fourth of the height of the at least one first side plate 7006cb and is smaller than or equal to two-thirds of the height of the at least one first side plate 7006cb.

In some embodiments of the present disclosure, the height of the at least one second side plate 7006bb is greater than or equal to one-fourth of the height of the at least one first side plate 7006cb and is smaller than or equal to three-fourths of the height of the at least one first side plate 7006cb.

In some embodiments of the present disclosure, the second region 72b is in shape of a rectangle or a square.

In above embodiments, the number of the detection electrode 71 is twenty-two, and the detection electrode 71 is arranged in two columns, with eleven of which in each column. In this way, an overall arrangement of the detection electrode 71 is in shape of a rectangle. Since the detection electrode 71 is disposed on the second region 72b, the second region 72b may have the shape matching with the arrangement of the detection electrode 71, i.e., the second region 72b may be in shape of a rectangle. In some embodiments, when the overall arrangement of the detection electrode 71 is in shape of a rectangle, the second region 72b may be in shape of a square. In some embodiments, when the overall arrangement of the detection electrode 71 is in shape of a square, the second region 72b may be in shape of a rectangle. In some embodiments, when the overall arrangement of the detection electrode 71 is in shape of a square, the second region 72b may be in shape of a square.

In some embodiments of the present disclosure, the groove 77 is in the shape of a cuboid or a cube.

Therefore, in above embodiment, the groove 77 may have a shape matching with the shape of the second region 72b. That is, in a case where the second region 72b is the rectangular region, the groove 77 may be a cuboid with a shape and a size that correspond to those of the second region 72b. In some embodiments, the shape of the groove 77 may be larger than the shape of the second region 72b. That is, in a case where the second region 72b is the rectangular region, the groove 77 may be a cuboid or a cube with a length and a width that are larger than a length or a width the second region 72b, as long as the detection electrode 71 in the second region 72b is located at the bottom surface 70062 of the groove 77.

In some embodiments of the present disclosure, the at least one first side plate 7006cb and the base 7006ca may be connected in one piece.

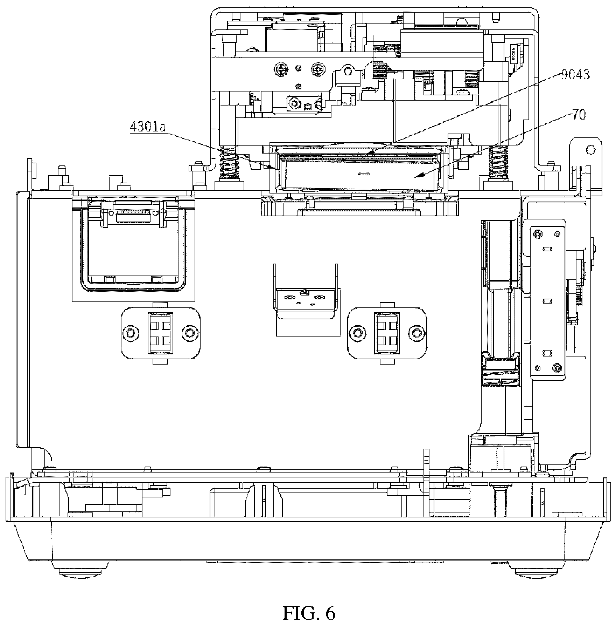

As shown in FIG. 6, FIG. 6 is a schematic structural view of a medical detection device according to some embodiments of the present disclosure. As shown in FIG. 6, the medical detection device includes a device body. The device body includes a slot 4301a and a contact electrode 9043. The slot 4301a is configured to receive the detection assembly 70. When the detection assembly 70 is inserted into the medical detection device, the contact electrode 9043 is in contact with the detection electrode 71 of the detection assembly 70.

Therefore, in above embodiments, the detection electrode 71 of the detection assembly 70 is located within the groove 77 defined by the recessed area of the top surface 70061 and the recessed area of the at least one side surface. When the detection assembly 70 is inserted into the slot of the medical detection device, the contact electrode in the medical detection device is driven to move toward the detection electrode 71, enabling the detection electrode 71 to be in contact with the contact electrode, thereby performing the sample detection. In addition, since the detection electrode 71 is disposed within the groove 77, the contact electrode in the medical detection device no longer has to avoid the detection electrode 71 on the top surface 70061 of the detection assembly 70. A distance between the contact electrode in the medical detection device and the detection electrode 71 on the detection assembly 70 may be designed to be smaller than the overall thickness of the detection assembly 70, thereby greatly reducing the dependence of the overall thickness of the detection assembly 70 on the size of the slot opening of the medical detection device.

Those skilled in the art may easily understand that many modifications and variations can be made to the apparatus and method while maintaining the teachings of the present disclosure. Therefore, the above disclosure should be considered as limited only by the scope of the appended claims.

## Claims

1. A detection assembly for a medical detection device, the detection assembly comprising:
a housing, comprising a top surface, a bottom surface, at least one side surface, a fluid inlet, and a fluid outlet, wherein the at least one side surface is connected to each of the top surface and the bottom surface, and a cavity is defined within the housing; and
a detection electrode;
wherein each of the fluid inlet and the fluid outlet is communicated with the cavity, a groove is recessed from an area of the top surface that is adjacent to the at least one side surface, the detection electrode is disposed in the groove, and the groove defined on a part of the top surface has an opening defined on the at least one side surface.

2. The detection assembly as claimed in claim 1, wherein the top surface and the bottom surface are arranged in parallel, the number of the at least one side surface is four, the four side surfaces are sequentially connected to each other, the groove and the opening are defined on an area where the top surface is connected to one of the four side surfaces, a width of the groove is smaller than a width of the top surface, and a width of the opening is smaller than a width of the one of the four side surfaces;
a portion of the groove corresponding to the top surface comprises a plane and a side surface, the plane is parallel to each of the top surface and the bottom surface, the side surface is perpendicular to the plane and is connected to each of the top surface and the plane, and the detection electrode is disposed on the plane.

3. The detection assembly as claimed in claim 1, wherein the housing further comprises a first housing and a second housing, the top surface is a surface of the first housing facing away from the second housing;
the detection assembly further comprises a circuit board with a first region and a second region, and the detection electrode is disposed on the second region;
the second housing comprises a base and at least one first side plate disposed around the base, a bottom end of the at least one first side plate is connected to an edge of base, a first notch is defined on an end of one of the at least one first side plate away from the base, an accommodating chamber is defined by the base and the at least one first side plate, the bottom surface is a surface of the base facing away from the first housing, and the at least one side surface is an outer surface of the at least one first side plate; and
the first housing cooperates with the at least one first side plate to cover the accommodating chamber, the circuit board is disposed between the base and the first housing, the first region is located within the accommodating chamber, and the second region is an area of the circuit board that is outside the accommodating chamber and corresponds to the groove, to form the plane of the groove.

4. The detection assembly as claimed in claim 3, wherein the first housing comprises a cover and at least one second side plate disposed around the cover, a top end of the at least one second side plate is connected to an edge of the cover, the top surface is a surface of the cover away from the second housing, a second notch is defined on an area of the cover that is close to the first notch, one of the at least one second side plate that corresponds to the second notch extends along a contour of the second notch;
the first notch, the second notch, the one of the at least one second side plate that corresponds to the second notch, and the second region together define the groove, the one of the at least one second side plate that corresponds to the second notch forms the side surface of the groove.

5. The detection assembly as claimed in claim 4, further comprising:
a third housing, comprising a mounting plate and at least one third side plate disposed around mounting plate, a bottom end of each of the at least one third side plate is connected to an edge of the mounting plate, each of the mounting plate and the at least one third side plate is disposed within the accommodating chamber, a top end of the at least one third side plate is connected to a top end of the at least one first side plate, the circuit board is disposed on the mounting plate and is located between the mounting plate and the first housing;
a third notch is defined on one of the at least one third side plate that corresponds to the first notch, and the third notch has a shape matching with a shape of the first notch.

6. The detection assembly as claimed in claim 5, wherein a raised rib is disposed on an edge of the mounting plate that is close to the at least one third side plate and is disposed around a periphery of the circuit board to limit the circuit board.

7. The detection assembly as claimed in claim 5, further comprising:
at least one fixing member;
wherein a first through hole is defined on the cover of the first housing, a second through hole is defined on the circuit board, a third through hole is defined on the mounting plate, a fourth through hole is defined on the base, and the at least one fixing member penetrates through the fourth through hole, the third through hole, the second through hole, and the first through hole, to fix the first housing, the circuit board, and the second housing to each other.

8. The detection assembly as claimed in claim 4, wherein a height of the at least one second side plate is greater than or equal to one-fourth of a height of the at least one first side plate and is smaller than or equal to two-thirds of the height of the at least one first side plate.

9. The detection assembly as claimed in claim 4, wherein a height of the at least one second side plate is greater than or equal to one-fourth of a height of the at least one first side plate and is smaller than or equal to three-fourths of the height of the at least one first side plate.

10. The detection assembly as claimed in claim 3, wherein the second region is in shape of a rectangle or a square.

11. The detection assembly as claimed in claim 3, wherein the at least one first side plate and the base are connected in one piece.

12. The detection assembly as claimed in claim 1, wherein the groove is in shape of a cuboid or a cube.

13. A medical detection device, comprising:
a device body, comprising a slot and a contact electrode, wherein the slot is configured to receive the detection assembly as claimed in one of claims 1-12; when the detection assembly is inserted into the medical detection device, the contact electrode is in contact with the detection electrode of the detection assembly.
